# EUROPÄISCHE PATENTANMELDUNG

(11) **EP 4 796 163 A1**
(43) Veröffentlichungstag der Anmeldung: **26.08.2026**
(21) Anmeldenummer: 26158766.1
(22) Anmeldetag: 16.02.2026
(51) Int. Cl.: A61N 1/36, A61B 5/0531, A61B 5/00, A61N 1/05

(54) **MEDIZINISCHES SYSTEM ZUR VERWENDUNG BEI EINER SCHMERZTHERAPIE**

(30) Priorität: 20.02.2025 DE 102025106366
(71) Anmelder: B. Braun Melsungen AG, 34212 Melsungen (DE)
(72) Erfinder: Wildhagen, Jens, 30659 Hannover (DE)
(74) Vertreter: Patentanwälte Ruff, Wilhelm, Beier, Dauster & Partner mbB

(57) **Zusammenfassung**

Die Erfindung betrifft ein medizinisches System zur Verwendung bei einer Schmerztherapie, aufweisend eine erste Hautelektrode und eine zweite Hautelektrode, die jeweils zur Applikation auf der Haut eines Patienten eingerichtet sind, wenigstens eine Invasivelektrode, die zur intrakorporalen Abgabe eines elektrischen Neurostimulationssignals eingerichtet ist, und eine Steuereinrichtung, die mit der ersten Hautelektrode, der zweiten Hautelektrode und der Invasivelektrode verbunden ist, wobei die Steuereinrichtung zum Ermitteln einer elektrischen Leitfähigkeit der Haut in Abhängigkeit von mittels der ersten Hautelektrode und der zweiten Hautelektrode erfassten und/oder abgegebenen elektrischen Messsignalen eingerichtet ist, wobei die ermittelte elektrische Leitfähigkeit ein Schmerzempfinden des Patienten repräsentiert, wobei die Steuereinrichtung zum Erzeugen des elektrischen Neurostimulationssignals eingerichtet ist, und wobei die Steuereinrichtung zum Anpassen des elektrischen Neurostimulationssignals in Abhängigkeit der ermittelten elektrischen Leitfähigkeit eingerichtet ist.

## Beschreibung

Die Erfindung betrifft ein medizinisches System zur Verwendung bei einer Schmerztherapie.

Elektrische Neurostimulation (kurz: Neurostimulation oder Neuromodulation) ist eine bekannte Therapieform zur Behandlung akuter oder chronischer Schmerzen. Dabei wird ein elektrisches Neurostimulationssignal mittels einer Invasivelektrode intrakorporal in unmittelbarer Nähe eines Nervs abgegeben, um dessen Leitungsverhalten für Schmerzimpulse zu unterdrücken und damit das Schmerzempfinden des Patienten zu lindern.

Weiter ist bekannt, dass das Schmerzempfinden über eine elektrische Leitfähigkeitsmessung ermittelt werden kann. Die Leitfähigkeitsmessung erfolgt üblicherweise unter Verwendung von Hautelektroden, die auf der Haut des Patienten appliziert werden, um die Leitfähigkeitsmessung durchzuführen. Ein hierfür geeignetes medizinisches System wird beispielsweise seitens des Unternehmens MedStorm am Markt angeboten.

Aufgabe der Erfindung ist es, ein medizinisches System bereitzustellen, das eine verbesserte Schmerztherapie ermöglicht.

Diese Aufgabe wird durch das Bereitstellen eines medizinischen Systems mit den Merkmalen des Anspruchs 1 gelöst. Vorteilhafte Weiterbildungen sind in den abhängigen Ansprüchen angegeben. Der Wortlaut der Ansprüche wird durch Bezugnahme zum Gegenstand der Beschreibung gemacht.

Das erfindungsgemäße medizinische System weist eine erste Hautelektrode, eine zweite Hautelektrode, wenigstens eine Invasivelektrode und eine Steuereinrichtung auf. Die erste Hautelektrode und die zweite Hautelektrode sind jeweils zur Applikation auf der Haut eines Patienten eingerichtet. Die wenigstens eine Invasivelektrode ist zur intrakorporalen Abgabe eines elektrischen Neurostimulationssignals eingerichtet. Die Steuereinrichtung ist mit der ersten Hautelektrode, der zweiten Hautelektrode und der Invasivelektrode verbunden. Die Steuereinrichtung ist zum Ermitteln einer elektrischen Leitfähigkeit der Haut in Abhängigkeit von mittels der ersten Hautelektrode und der zweiten Hautelektrode erfassten und/oder abgegebenen elektrischen Messsignalen eingerichtet, wobei die ermittelte elektrische Leitfähigkeit ein Schmerzempfinden des Patienten repräsentiert. Weiter ist die Steuereinrichtung zum Erzeugen des elektrischen Neurostimulationssignals eingerichtet. Weiter ist die Steuereinrichtung zum Anpassen des elektrischen Neurostimulationssignals in Abhängigkeit der ermittelten elektrischen Leitfähigkeit und damit des Schmerzempfindens des Patienten eingerichtet. Durch das erfindungsgemäße medizinische System kann eine verbesserte Schmerztherapie erreicht werden, indem das mittels der Invasivelektrode zwecks Schmerzlinderung abzugebende elektrische Neurostimulationssignal in Abhängigkeit des aktuellen Schmerzempfindens des Patienten angepasst wird. Mit anderen Worten: Das erfindungsgemäße medizinische System erlaubt eine an den Schmerz des Patienten angepasste/optimierte Neurostimulation. Mit nochmals anderen Worten: Das erfindungsgemäße medizinische System erlaubt eine Regelung des elektrischen Neurostimulationssignals in Abhängigkeit des ermittelten Schmerzempfindens. Das besagte Anpassen kann auch als Regeln, Einregeln verstanden werden. Es besteht folglich ein Feedback-Loop zwischen der ermittelten elektrischen Leitfähigkeit und dem elektrischen Neurostimulationssignal. Die erste Hautelektrode und die zweite Hautelektrode können jede für den vorliegenden Zweck geeignete Gestaltung aufweisen. Die erste Hautelektrode und die zweite Hautelektrode sind zum Erfassen und/oder Abgeben der für die elektrische Leitfähigkeitsmessung erforderlichen elektrischen Messsignale eingerichtet. Die wenigstens eine Invasivelektrode kann jede für den vorliegenden Zweck geeignete Gestaltung aufweisen. Die wenigstens eine Invasivelektrode ist zum Einführen in den menschlichen und/oder tierischen Körper eingerichtet. Bei einer Ausgestaltung ist die Invasivelektrode ein Elektrodendraht (Lead). Bei einer weiteren Ausgestaltung ist die wenigstens eine Invasivelektrode an einer Invasivkomponente angeordnet, bei der es sich beispielsweise um eine Kanüle, einen Katheter oder dergleichen handelt. Das elektrische Neurostimulationssignal ist dazu eingerichtet, das Leitungsverhalten eines Nervs für Schmerzimpulse zu unterdrücken. Hierfür geeignete elektrische Signalarten und/oder -formen sind dem Fachmann bekannt. Zudem ist der physikalisch/physiologische Zusammenhang zwischen elektrischer Leitfähigkeit der Haut und dem Schmerzempfinden des Patienten grundsätzlich bekannt. Auf weitere Details hierzu muss deshalb an dieser Stelle nicht eingegangen werden.

In weiterer Ausgestaltung der Erfindung ist die Steuereinrichtung derart zum Anpassen des elektrischen Neurostimulationssignals eingerichtet, dass das Schmerzempfinden des Patienten verringert, vorzugsweise minimiert, wird. Bei dieser Ausgestaltung der Erfindung wird das elektrische Neurostimulationssignal - in Abhängigkeit der ermittelten elektrischen Leitfähigkeit - über der Zeit so lange angepasst, bis die sich infolge des elektrischen Neurostimulationssignals einstellende geänderte elektrische Leitfähigkeit einem verringerten, vorzugsweise minimierten, Schmerzempfinden entspricht.

In weiterer Ausgestaltung der Erfindung ist die erste Hautelektrode dazu eingerichtet, als Gegenelektrode der wenigstens einen Invasivelektrode bei der Abgabe des elektrischen Neurostimulationssignals zu fungieren. Bei dieser Ausgestaltung hat die erste Hautelektrode folglich zwei Funktionen. Zum einen dient die erste Hautelektrode dem Erfassen und/oder Abgeben der elektrischen Messsignale zur Ermittlung der elektrischen Leitfähigkeit. Zum anderen dient die Hautelektrode als Gegenelektrode für die wenigstens eine Invasivelektrode. Hierdurch kann auf eine weitere, ausschließlich als Gegenelektrode fungierende Elektrode verzichtet werden. Hierdurch wird der Aufbau des medizinischen Systems vereinfacht. Zudem ergibt sich eine vereinfachte Applikation am Patienten, da auf das Anlegen einer gesonderten Gegenelektrode verzichtet werden kann.

In weiterer Ausgestaltung der Erfindung sind die erste Hautelektrode und die zweite Hautelektrode jeweils dazu eingerichtet, als Gegenelektrode der wenigstens einen Invasivelektrode bei der Abgabe des elektrischen Neurostimulationssignals zu fungieren.

In weiterer Ausgestaltung der Erfindung ist die Steuereinrichtung dazu eingerichtet, das elektrische Neurostimulationssignal und die elektrischen Messsignale im Zeitmultiplex zu verarbeiten. Im Zeitmultiplex meint, dass die betreffenden Signale über der Zeit abwechselnd verarbeitet werden, vorzugsweise über ein und dieselbe Signalleitung. Hierdurch kommt auch der Steuereinrichtung eine Doppelfunktion zu. Zum einen dient die Steuereinrichtung der Ermittlung des Schmerzempfindens, wobei das Schmerzempfinden mittelbar über die elektrische Leitfähigkeitsmessung ermittelt wird. Zum anderen dient die Steuereinrichtung zum Erzeugen und Anpassen des elektrischen Neurostimulationssignals.

Das Anpassen des elektrischen Neurostimulationssignals erfolgt über eine Anpassung/Änderung einzelner oder mehrerer Parameter des Neurostimulationssignals. Die Steuereinrichtung ist dementsprechend eingerichtet. Bei unterschiedlichen Ausgestaltungen werden unterschiedliche Parameter des elektrischen Neurostimulationssignals geändert/angepasst.

In weiterer Ausgestaltung der Erfindung ist die Steuereinrichtung zum Anpassen einer Stromstärke des elektrischen Neurostimulationssignals in Abhängigkeit der ermittelten elektrischen Leitfähigkeit eingerichtet. Bei einer Ausgestaltung wird die Stromstärke erhöht, sobald das ermittelte Schmerzempfinden zunimmt. Bei einer Ausgestaltung wird die Stromstärke verringert, sobald das ermittelte Schmerzempfinden abnimmt. Bei einer Ausgestaltung ist eine hierzu umgekehrte Anpassung der Stromstärke vorgesehen.

In weiterer Ausgestaltung der Erfindung ist die Steuereinrichtung dazu eingerichtet, eine Modulationsfrequenz des elektrischen Neurostimulationssignals in Abhängigkeit der ermittelten elektrischen Leitfähigkeit anzupassen. Bei einer Ausgestaltung wird die Modulationsfrequenz erhöht, sobald das ermittelte Schmerzempfinden zunimmt. Bei einer Ausgestaltung wird die Modulationsfrequenz verringert, sobald das ermittelte Schmerzempfinden abnimmt. Bei einer Ausgestaltung ist eine hierzu umgekehrte Anpassung der Modulationsfrequenz vorgesehen.

In weiterer Ausgestaltung der Erfindung ist die Steuereinrichtung dazu eingerichtet, eine Pulsweite des elektrischen Neurostimulationssignals in Abhängigkeit der ermittelten elektrischen Leitfähigkeit anzupassen. Bei einer Ausgestaltung wird die Pulsweite erhöht, sobald das ermittelte Schmerzempfinden zunimmt. Bei einer Ausgestaltung wird die Pulsweite verringert, sobald das ermittelte Schmerzempfinden abnimmt. Bei einer Ausgestaltung ist eine hierzu umgekehrte Anpassung der Pulsweite vorgesehen.

In weiterer Ausgestaltung der Erfindung ist die Steuereinrichtung zum Anpassen einer Signalform des elektrischen Neurostimulationssignals in Abhängigkeit der ermittelten elektrischen Leitfähigkeit eingerichtet. Bei dieser Ausgestaltung der Erfindung erlaubt die Steuereinrichtung, das elektrische Neurostimulationssignal in unterschiedlichen Signalformen zu erzeugen. Je nach Höhe des ermittelten Schmerzempfindens kann die Signalform angepasst werden, indem eine spezifische Signalform aus mehreren unterschiedlichen Signalformen ausgewählt und erzeugt wird. Als Signalformen denkbar sind beispielsweise Impulse, eine Abfolge von Impulsen gefolgt von einer vorgegebenen Pause (burst), ein Sinussignal, ein Rechtecksignal, ein Dreiecks- oder Sägezahnsignal oder dergleichen.

In weiterer Ausgestaltung der Erfindung weist das medizinische System zudem wenigstens eine weitere Invasivelektrode auf, wobei die Steuereinrichtung dazu eingerichtet ist, die Invasivelektrode und die weitere Invasivelektrode in Abhängigkeit der ermittelten elektrischen Leitfähigkeit wahlweise anzusteuern. Durch das wahlweise Ansteuern wird das elektrische Stimulationssignal entweder über die Invasivelektrode oder die weitere Invasivelektrode oder beide Invasivelektroden abgegeben. Hierdurch kann unter anderem die Position der Signalabgabe angepasst werden. Bei einer Ausgestaltung sind die Invasivelektrode und die weitere Invasivelektrode jeweils an einer Invasivkomponente angeordnet, im Speziellen entlang einer Längsachse der Invasivkomponente voneinander beabstandet.

Die Erfindung betrifft zudem ein Verfahren zur Verwendung bei einer Schmerztherapie. Das erfindungsgemäße Verfahren weist die Schritte auf: Ermitteln einer elektrischen Leitfähigkeit der Haut eines Patienten, wobei die elektrische Leitfähigkeit unter Verwendung von Messsignalen einer ersten Hautelektrode und einer zweiten Hautelektrode, die jeweils auf der Haut des Patienten appliziert sind, und mittels einer mit den Hautelektroden verbundenen Steuereinrichtung ermittelt wird; Erzeugen und Abgeben eines elektrischen Neurostimulationssignals, wobei das elektrische Neurostimulationssignal mittels der Steuereinrichtung erzeugt und mittels einer Invasivelektrode intrakorporal abgegeben wird; Anpassen des elektrischen Neurostimulationssignals, wobei das elektrische Neurostimulationssignal mittels der Steuereinrichtung und in Abhängigkeit der ermittelten elektrischen Leitfähigkeit und damit des Schmerzempfindens des Patienten angepasst wird. Das erfindungsgemäße medizinische System und seine Ausgestaltungen sind zum Ausführen des erfindungsgemäßen Verfahrens eingerichtet. Umgekehrt kann das erfindungsgemäße Verfahren unter Verwendung des medizinischen Systems und dessen Ausgestaltungen ausgeführt werden. Vorteile und weitere Merkmale des erfindungsgemäßen Verfahrens ergeben sich unmittelbar und eindeutig aus der Offenbarung des erfindungsgemäßen medizinischen Systems und seinen Ausgestaltungen, auf welche ausdrücklich verwiesen und Bezug genommen wird.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.

Es zeigen:
- Fig. 1: in schematischer Blockdarstellung eine Ausführungsform eines erfindungsgemäßen medizinischen Systems zur Verwendung bei einer Schmerztherapie und
- Fig. 2: in schematischer Blockdarstellung eine Ausführungsform eines erfindungsgemäßen Verfahrens zur Verwendung bei einer Schmerztherapie.

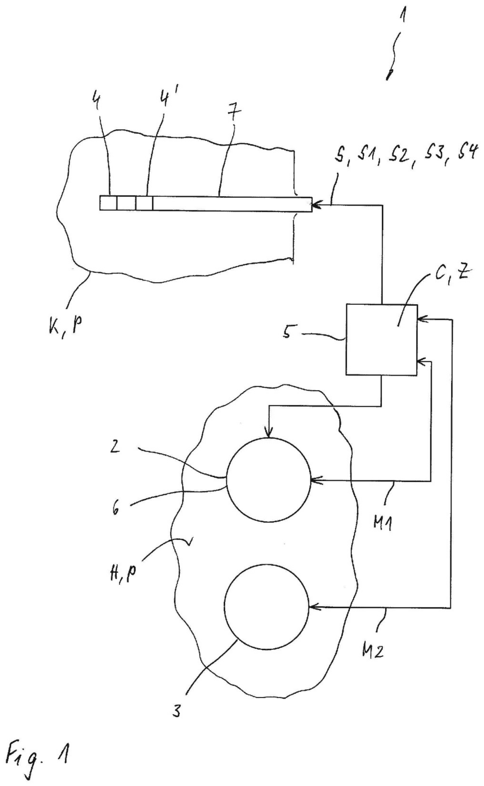

Gemäß Fig. 1 ist ein medizinisches System 1 zur Verwendung bei einer Schmerztherapie vorgesehen. Das medizinische System 1 erlaubt eine an das aktuelle Schmerzempfinden eines Patienten angepasste Neurostimulation.

Das medizinische System 1 weist eine erste Hautelektrode 2, eine zweite Hautelektrode 3, wenigstens eine Invasivelektrode 4 und eine Steuereinrichtung 5 auf.

Die erste Hautelektrode 2 und die zweite Hautelektrode 3 sind jeweils zur Applikation auf der Haut H eines Patienten P eingerichtet. Dabei ist die in Fig. 1 schematisch gezeigte Gestaltung und Anordnung der beiden Hautelektroden 2, 3 als rein exemplarisch zu verstehen.

Die wenigstens eine Invasivelektrode ist zur intrakorporalen Abgabe eines elektrischen Neurostimulationssignals S eingerichtet. Wirkt das Neurostimulationssignal S auf einen Nerv, wird dessen Fähigkeit zur Weiterleitung elektrischer Schmerzimpulse beeinträchtigt, wodurch der Patient P weniger Schmerzen verspürt.

Bei der gezeigten Ausführungsform ist die Invasivelektrode 4 an einer Invasivkomponente 7 angeordnet. Die Invasivkomponente 7 ist zum Einführen in den Körper P des Patienten eingerichtet und beispielsweise als Kanüle, Katheter oder dergleichen gestaltet.

Bei einer in den Figuren nicht gezeigten Ausführungsform ist die Invasivelektrode als Elektrodendraht zum Einführen in den Körper K des Patienten P ausgeführt.

Bei der gezeigten Ausführungsform weist das medizinische System 1 zudem eine optionale weitere Invasivelektrode 4' auf. Die weitere Invasivelektrode 4' ist nicht bei sämtlichen Ausführungsformen vorhanden.

Vorliegend sind die Invasivelektrode 4 und die weitere Invasivelektrode 4' im Bereich eines distalen Endes der Invasivkomponente 7 angeordnet und proximodistal voneinander beabstandet.

Die Steuereinrichtung 5 ist mit der ersten Hautelektrode 2, der zweiten Hautelektrode 3 und der Invasivelektrode 4 verbunden. Bei der gezeigten Ausführungsform ist die Steuereinrichtung 5 zudem mit der weiteren Invasivelektrode 4' verbunden.

Die Steuereinrichtung 5 ist zum Ermitteln einer elektrischen Leitfähigkeit C der Haut H eingerichtet. Das Ermitteln der elektrischen Leitfähigkeit C erfolgt vorliegend in Abhängigkeit von mittels der ersten Hautelektrode 2 und der zweiten Hautelektrode 3 erfassten und/oder abgegebenen elektrischen Messsignalen M1, M2.

Die mittels der Steuereinrichtung 5 und den beiden Hautelektroden 2, 3 ermittelte elektrische Leitfähigkeit C der Haut H korreliert mit einem Schmerzempfinden Z des Patienten P. Mit anderen Worten: Die Ermittlung der elektrischen Leitfähigkeit C erlaubt einen Rückschluss auf das Schmerzempfinden Z, d. h. einen Grad der seitens des Patienten P aktuell empfundenen Schmerzen.

Weiter ist die Steuereinrichtung 5 zum Erzeugen des mittels der wenigstens einen Invasivelektrode 4 abgebbaren elektrischen Neurostimulationssignals S eingerichtet.

Weiter ist die Steuereinrichtung 5 zum Anpassen des elektrischen Neurostimulationssignals S in Abhängigkeit der ermittelten elektrischen Leitfähigkeit C und damit auch des Schmerzempfindens Z des Patienten P eingerichtet. Mit anderen Worten: Die Steuereinrichtung 5 erlaubt eine Regelung des Neurostimulationssignals S in Abhängigkeit des Schmerzempfindens Z.

Bei der gezeigten Ausführungsform wird das elektrische Neurostimulationssignal S derart angepasst, dass das Schmerzempfinden Z des Patienten verringert wird. Im besten Fall wird eine Minimierung erreicht. Die Steuereinrichtung 5 ist dementsprechend eingerichtet.

Bei der gezeigten Ausführungsform fungiert die erste Hautelektrode 2 als Gegenelektrode 6 für die wenigstens eine Invasivelektrode 4 (und/oder Invasivelektrode 4') bei der Abgabe des elektrischen Neurostimulationssignals S. Diese zusätzliche Funktion der ersten Hautelektrode 2 als Gegenelektrode 6 wird in Fig. 1 durch eine weitere zwischen der Steuereinrichtung 5 und der ersten Hautelektrode 2/Gegenelektrode 6 erstreckte Signalverbindung verdeutlicht.

Bei einer in den Figuren nicht gezeigten Ausführungsform sind beide Hautelektroden dazu eingerichtet, als Gegenelektrode zu fungieren.

Bei der gezeigten Ausführungsform ist die Steuereinrichtung 5 dazu eingerichtet, das elektrische Neurostimulationssignal S und die elektrischen Messsignale M1, M2 im Zeitmultiplex zu verarbeiten. Hierdurch ergeben sich weitere Vorteile.

Zwecks Anpassung des elektrischen Neurostimulationssignals S ist die Steuereinrichtung 5 bei der gezeigten Ausführungsform dazu eingerichtet, unterschiedliche Parameter S1, S2, S3, S4 des elektrischen Neurostimulationssignals S zu verändern. Diese Veränderung erfolgt in Abhängigkeit der ermittelten elektrischen Leitfähigkeit C. Bei den besagten Parametern S1, S2, S3, S4 handelt es sich vorliegend um eine Stromstärke S1, eine Modulationsfrequenz S2, eine Pulsweite S3 und eine Signalform S4 des elektrischen Neurostimulationssignals S.

Die besagten Parameter S1, S2, S3, S4 können jeweils einzeln oder in beliebiger Kombination miteinander verändert/angepasst werden.

Bei der gezeigten Ausführungsform ist die Steuereinrichtung 5 zudem dazu eingerichtet, die Invasivelektrode 4 und die weitere Invasivelektrode 4' in Abhängigkeit der ermittelten elektrischen Leitfähigkeit C wahlweise anzusteuern. Je nach aktuellem Schmerzempfinden Z des Patienten P kann das Neurostimulationssignal S folglich entweder mittels der Invasivelektrode 4 oder der weiteren Invasivelektrode 4' oder mittels beider Invasivelektroden 4, 4' abgegeben werden.

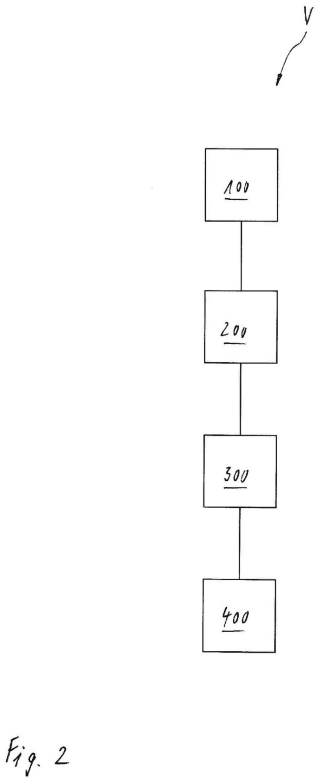

Das in Fig. 2 schematisch vereinfacht dargestellte Verfahren V kann unter Verwendung des medizinischen Systems 1 nach Fig. 1 ausgeführt werden. Es sieht Schritte 100, 200, 300 und 400 vor.

In dem Schritt 100 wird die elektrische Leitfähigkeit C der Haut H des Patienten P ermittelt. Die Ermittlung erfolgt unter Verwendung der Messsignale M1, M2 der ersten Hautelektrode 2 und der zweiten Hautelektrode 3, wobei diese während des Verfahrens V jeweils auf der Haut H appliziert sind. Die Ermittlung erfolgt mittels der Steuereinrichtung 5, die mit den beiden Hautelektroden 2, 3 verbunden ist.

Der Schritt 200 sieht das Erzeugen des elektrischen Neurostimulationssignals S mittels der Steuereinrichtung 5 vor.

Der Schritt 300 sieht das Abgeben des mittels der Steuereinrichtung 5 erzeugten elektrischen Neurostimulationssignals S mittels der Invasivelektrode 4 und/oder der weiteren Invasivelektrode 4' vor. Die Abgabe erfolgt intrakorporal, also im Körper K des Patienten P.

Der Schritt 400 sieht ein Anpassen des elektrischen Neurostimulationssignals S in Abhängigkeit der im Schritt 100 ermittelten elektrischen Leitfähigkeit C vor. Das Anpassen erfolgt wiederum mittels der Steuereinrichtung 5.

## Patentansprüche

1. Medizinisches System (1) zur Verwendung bei einer Schmerztherapie, aufweisend
eine erste Hautelektrode (2) und eine zweite Hautelektrode (3), die jeweils zur Applikation auf der Haut (H) eines Patienten (P) eingerichtet sind,
wenigstens eine Invasivelektrode (4), die zur intrakorporalen Abgabe eines elektrischen Neurostimulationssignals (S) eingerichtet ist, und
eine Steuereinrichtung (5), die mit der ersten Hautelektrode (2), der zweiten Hautelektrode (3) und der Invasivelektrode (4) verbunden ist,
wobei die Steuereinrichtung (5) zum Ermitteln einer elektrischen Leitfähigkeit (C) der Haut (H) in Abhängigkeit von mittels der ersten Hautelektrode (2) und der zweiten Hautelektrode (3) erfassten und/oder abgegebenen elektrischen Messsignalen (M1, M2) eingerichtet ist, wobei die ermittelte elektrische Leitfähigkeit (C) ein Schmerzempfinden (Z) des Patienten (P) repräsentiert,
wobei die Steuereinrichtung (5) zum Erzeugen des elektrischen Neurostimulationssignals (S) eingerichtet ist,
und wobei die Steuereinrichtung (5) zum Anpassen des elektrischen Neurostimulationssignals (S) in Abhängigkeit der ermittelten elektrischen Leitfähigkeit (C) eingerichtet ist.

2. Medizinisches System (1) nach Anspruch 1, wobei die Steuereinrichtung (5) derart zum Anpassen des elektrischen Neurostimulationssignals (5) eingerichtet ist, dass das Schmerzempfinden (Z) des Patienten (P) verringert, vorzugsweise minimiert, wird.

3. Medizinisches System (1) nach Anspruch 1 oder 2, wobei die erste Hautelektrode (2) dazu eingerichtet ist, als Gegenelektrode (6) der wenigstens einen Invasivelektrode (4) bei der Abgabe des elektrischen Neurostimulationssignals (S) zu fungieren.

4. Medizinisches System (1) nach einem der vorhergehenden Ansprüche, wobei die erste Hautelektrode (2) und die zweite Hautelektrode (3) jeweils dazu eingerichtet sind, als Gegenelektrode (6) der wenigstens einen Invasivelektrode (4) bei der Abgabe des elektrischen Neurostimulationssignals (S) zu fungieren.

5. Medizinisches System (1) nach Anspruch 3 oder 4, wobei die Steuereinrichtung (5) dazu eingerichtet ist, das elektrische Neurostimulationssignal (S) und die elektrischen Messsignale (M1, M2) im Zeitmultiplex zu verarbeiten.

6. Medizinisches System (1) nach einem der vorhergehenden Ansprüche, wobei die Steuereinrichtung (5) eingerichtet ist zum Anpassen einer Stromstärke (S1) des elektrischen Neurostimulationssignals (S) in Abhängigkeit der ermittelten elektrischen Leitfähigkeit (C).

7. Medizinisches System (1) nach einem der vorhergehenden Ansprüche, wobei die Steuereinrichtung (5) eingerichtet ist zum Anpassen einer Modulationsfrequenz (S2) des elektrischen Neurostimulationssignals (S) in Abhängigkeit der ermittelten elektrischen Leitfähigkeit (C).

8. Medizinisches System (1) nach einem der vorhergehenden Ansprüche, wobei die Steuereinrichtung (5) eingerichtet ist zum Anpassen einer Pulsweite (S3) des elektrischen Neurostimulationssignals (S) in Abhängigkeit der ermittelten elektrischen Leitfähigkeit (C).

9. Medizinisches System (1) nach einem der vorhergehenden Ansprüche, wobei die Steuereinrichtung (5) eingerichtet ist zum Anpassen einer Signalform (S4) des elektrischen Neurostimulationssignals (S) in Abhängigkeit der ermittelten elektrischen Leitfähigkeit (C).

10. Medizinisches System (1) nach einem der vorhergehenden Ansprüche, weiter aufweisend wenigstens eine weitere Invasivelektrode (4'), wobei die Steuereinrichtung (5) eingerichtet ist zum wahlweisen Ansteuern der Invasivelektrode (4) und der weiteren Invasivelektrode (4') in Abhängigkeit der ermittelten elektrischen Leitfähigkeit (C).

11. Verfahren (V) zur Verwendung bei einer Schmerztherapie, aufweisend die Schritte:
Ermitteln (100) einer elektrischen Leitfähigkeit (C) der Haut (H) eines Patienten (P), wobei die elektrische Leitfähigkeit (C) unter Verwendung von Messsignalen (M1, M2) einer ersten Hautelektrode (2) und einer zweiten Hautelektrode (3), die jeweils auf der Haut (H) des Patienten (P) appliziert sind, und mittels einer mit den Hautelektroden (2, 3) verbundenen Steuereinrichtung (5) ermittelt wird,
Erzeugen (200) und Abgeben (300) eines elektrischen Neurostimulationssignals (S), wobei das elektrische Neurostimulationssignal (S) mittels der Steuereinrichtung (5) erzeugt und mittels einer Invasivelektrode (4) intrakorporal abgegeben wird,
Anpassen (400) des elektrischen Neurostimulationssignals (S), wobei das elektrische Neurostimulationssignal (S) mittels der Steuereinrichtung (5) und in Abhängigkeit der ermittelten elektrischen Leitfähigkeit (C) angepasst wird.
